# EUROPEAN PATENT APPLICATION

(11) **EP 2 315 035 A2**
(43) Date of publication of application: **27.04.2011**
(21) Application number: 10013715.7
(22) Date of filing: 18.10.2010
(51) Int. Cl.: G01N 35/00

(54) **Sample processing unit and sample processing method**

(30) Priority: 20.10.2009 JP 2009241490; 18.05.2010 JP 2010114628
(71) Applicant: Aoi Seiki Co., Ltd, Kumamoto-ken (JP)
(72) Inventor: Itoh, Teruaki, Kumamoto-shi Kumamoto-ken (JP)
(74) Representative: Plöger, Jan Manfred

(57) **Abstract**

An example of the invention is a sample processing unit (1) having, a subdivision/dispensing section (35) including a nozzle needle (53) configured to be able to penetrate a stopper body (26) fitted in a sample container (25) in which a sample can be contained, and configured to subject the sample container (25) or the sample to processing, a detection unit (34) configured to detect a state of the sample or the sample container (25) at the time of the processing, and a label removal unit (33) configured to remove at least part of a label attached to a side part of the sample container (25) prior to the detection.

## Description

The present invention relates to a sample processing unit and sample processing method configured to carry out processing in a sample container in which a sample is contained.

As a sample processing apparatus configured to carry out processing in a sample container containing therein a sample, an analysis apparatus or the like configured to extract a blood serum separated from blood acquired by, for example, blood drawing through a centrifugal separation process as a sample, and carry out analysis processing by infusing a reagent into the sample to detect the reaction is known (see, for example, Jpn. Pat. Appln. KOKAI Publication No. 2008-76185). A test tube configured to contain therein a sample such as blood is made of, for example, transparent glass or plastic, is formed into a cylindrical shape having an opening at an upper part thereof, and is configured to contain a sample therein. A stopper body constituted of rubber or synthetic resin material is fitted into the opening at an upper end of the test tube. In a general sample processing apparatus, a tube opening process for removing the stopper body closing the upper opening of the test tube is carried out prior to the sample processing such as subdivision/dispensing or the like and, in a state where the upper part of the test tube is opened, an instrument such as a nozzle or the like is inserted into the test tube from the opening to subject the sample inside to sample processing such as subdivision/dispensing or the like. In the sample processing apparatus configured to carry out, prior to the sample processing, the tube opening processing to open the test tube, and thereafter carry out the sample processing in the manner described above, there is the problem that a lot of time is required for the tube opening processing, and the processing efficiency is poor. Further, there is the possibility of the sample dispersing or evaporating from the opening part at the upper part of the test tube.

In consideration of these circumstances, means for carrying out processing by using a member having a sharp needle-like shape, and penetrating through the stopper body into the sample container is conceivable.

However, the above technique involves the following problem. That is, there is the problem that a label is attached to the side part of the sample container in some cases, hence it is difficult to detect the state inside the sample in the stopper-inserted state, and the processing accuracy is lowered.

An object of the present invention is to provide a sample processing unit and sample processing method which are capable of processing a high degree of detection accuracy in the stopper-inserted state.

An aspect of the invention is a sample processing unit comprising, a penetration processing unit including a penetration insertion section configured to be able to penetrate a stopper body fitted in a sample container in which a sample can be contained, and configured to subject the sample container or the sample to processing, a detection unit configured to detect a state of the sample or the sample container at the time of the processing, and, a label removal unit configured to remove at least part of a label attached to a side part of the sample container prior to the detection.

An another aspect of the invention is a sample processing unit comprising, the sample container is a test tube at least part of which is transparent, the stopper body is constituted of a material including rubber or synthetic resin material, and the penetration processing unit is a subdivision/dispensing unit including a nozzle needle comprising a sharp distal end, and configured to carry out subdivision processing or dispensing processing of the sample in a state where the distal end of the nozzle needle is inserted into the sample container penetratingly through the stopper body.

An another aspect of the invention is a sample processing unit comprising, the detection unit acquires image data on the sample or the sample container, and detects at least any one of positions of surfaces of a blood serum, silicone, and a blood clot all of which serve as constituents of the sample contained in the sample container, and the penetration insertion section on the basis of the image data.

An another aspect of the invention is a sample processing unit comprising, a transfer unit configured to transfer the sample container along a predetermined transfer path while holding the sample container upright with the stopper body arranged on the upper side, wherein the penetration processing unit and the detection unit are arranged along the transfer path, and the label removal unit is arranged on the upstream side of the penetration processing unit on the transfer path.

An another aspect of the invention is a sample processing unit comprising, the penetration processing unit further comprises a chip slope including a tubular section comprising a cavity with a diameter larger than the nozzle needle, and configured to penetrate the stopper body, and a diameter-expansion section which continues from the tubular section, and the diameter of which is gradually expanded, the chip slope is inserted into the sample container penetratingly through the stopper body prior to the subdivision processing of the sample, and the subdivision processing is carried out in a state where the inside and the outside of the sample container communicate with each other through the cavity of the chip slope.

An another aspect of the invention is a sample processing unit comprising, a cutter configured to form an opening in the stopper body, wherein an opening is formed in the stopper body by using the cutter prior to the subdivision processing of the sample.

An another aspect of the invention is a sample processing method of carrying out processing in a sample container in which a sample can be contained comprising, a step of causing a penetration insertion section capable of penetrating a stopper body fitted in the sample container to penetrate the stopper body to insert the penetration insertion section into the sample container; and a step of carrying out processing in a state where the penetration insertion section is inserted in the sample container.

In the sample processing unit and sample processing method according to the present invention, which can process a detection in the stopper-inserted state.

The invention can be more fully understood from the following detailed description when taken in conjunction with the accompanying drawings, in which:
FIG. 1 is an explanatory view of a sample processing unit according to a first embodiment of the present invention.
FIG. 2 is a plan view of a subdivision/dispensing apparatus according to the first embodiment.
FIG. 3 is a side view of the subdivision/dispensing apparatus according to the first embodiment.
FIGS. 4A and 4B are side views of a label exfoliation section according to the first embodiment.
FIGS. 5A, 5B, 5C, and 5D are explanatory views of a structure and subdivision processing of a subdivision/dispensing section according to the first embodiment.
FIG. 6 is an explanatory view of the subdivision/dispensing apparatus according to the first embodiment.
FIGS. 7A, 7B, 7C, and 7D are explanatory views of a structure and subdivision processing of a subdivision/dispensing section according to a second embodiment of the present invention.
FIG. 8 is a plan view of a chip slope according to the second embodiment.
FIGS. 9A, 9B, and 9C are plan views of a stopper body according to a third embodiment of the present invention.
FIGS. 10A, 10B, 10C, and 10D are explanatory views of a structure and subdivision processing of a subdivision/dispensing section according to the third embodiment.

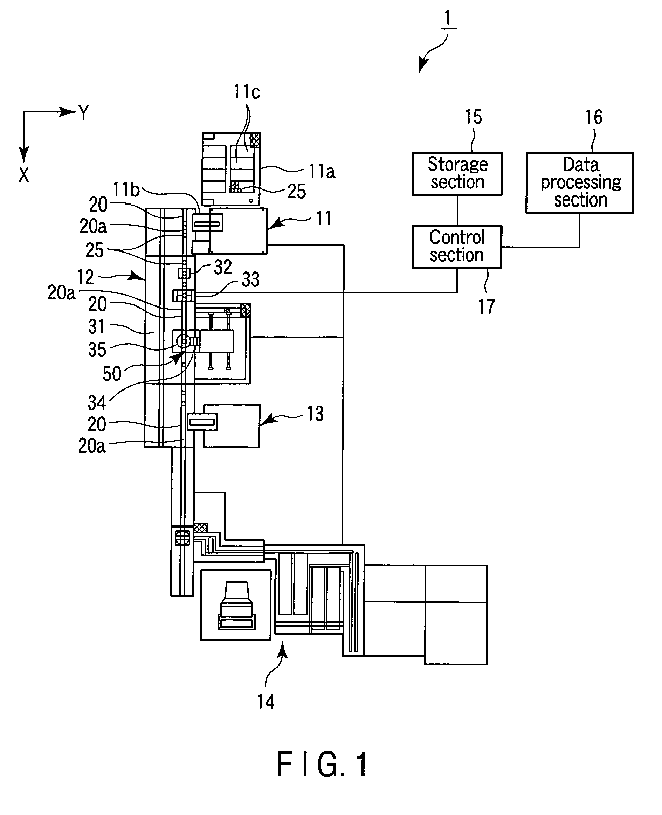

Hereinafter, a sample processing unit 1 serving as a sample processing apparatus according to an embodiment of the present invention will be described below with reference to FIGS. 1 to 5D. It should be noted that in each of FIGS. 1 to 5D, the configuration is shown by appropriately enlarging, reducing, or abbreviating the drawing. Arrows X, Y, and Z in each of FIGS. 1 to 5D, respectively show three directions perpendicular to each other. It should be noted that here the case where the X-, Y-, and Z-axes are set in the transfer path direction, width direction of the transfer path, and vertical direction, respectively, is shown.

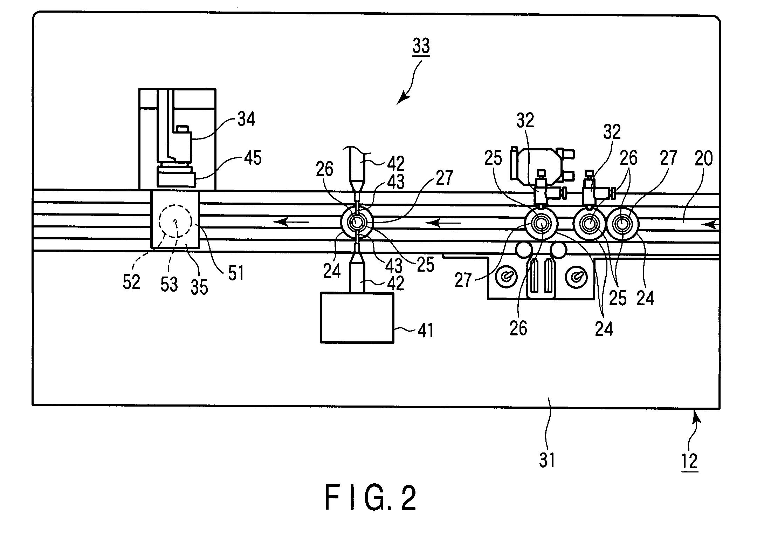

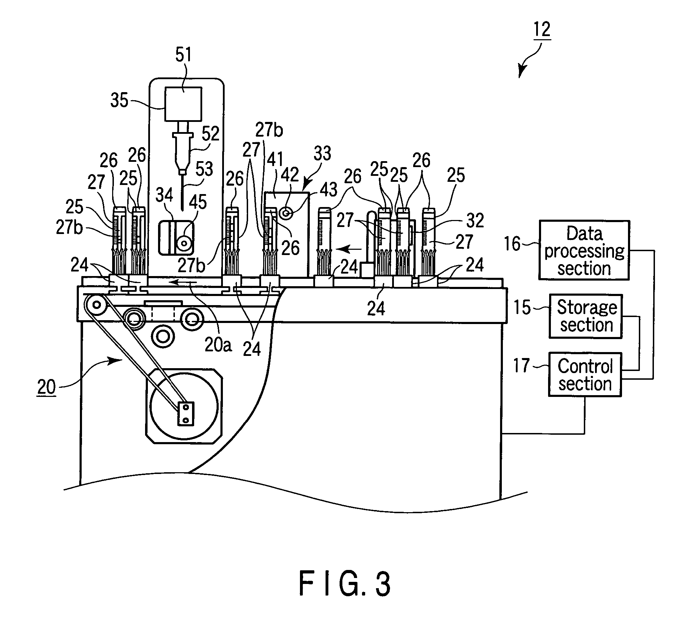

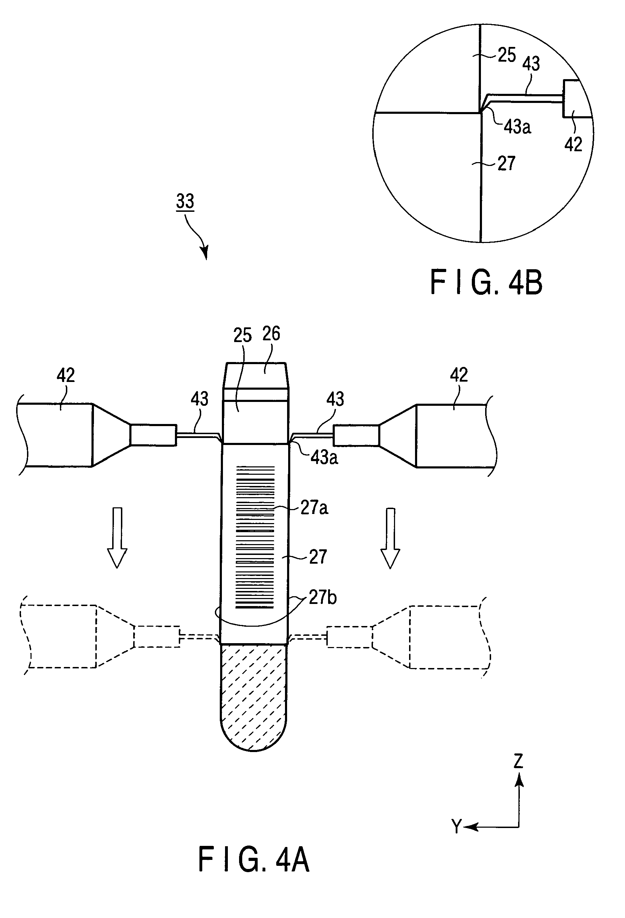

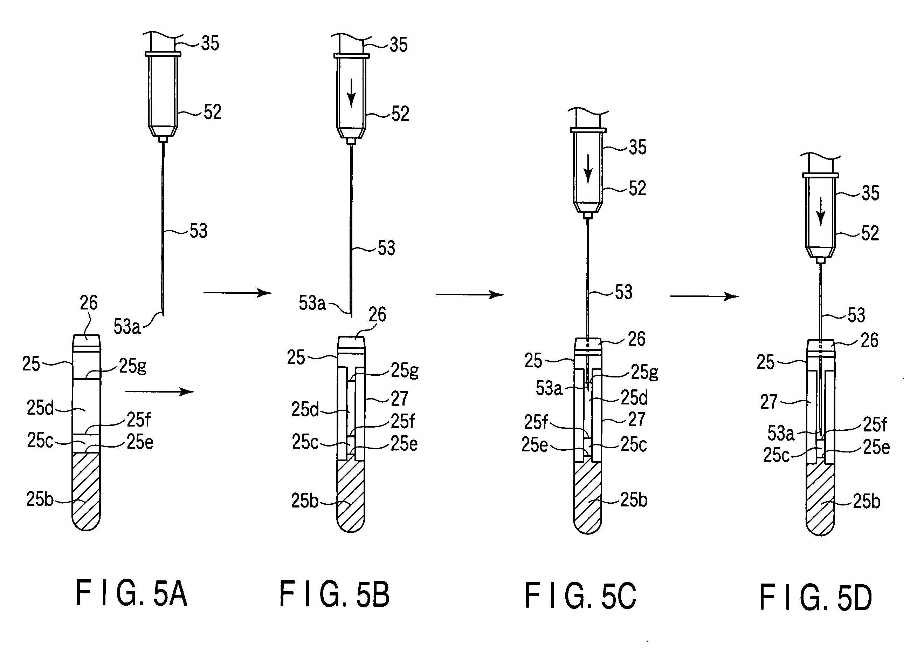

FIG. 1 is an explanatory view of a sample processing unit. FIGS. 2 and 3 are a plan view and side view of a subdivision/dispensing apparatus. FIGS. 4A and 4B are side views of a label exfoliation unit. FIGS. 5A to 5D are explanatory views showing a structure and subdivision processing of a subdivision/dispensing unit.

As shown in FIG. 1, the sample processing unit 1 is constituted by being provided with, in sequence from the upstream side of a transfer path 20a, a carry-in apparatus 11, subdivision/dispensing apparatus 12, carry-out apparatus 13, analysis apparatus 14, and is further provided with storage section 15 (storage means) configured to store therein various data items, data processing section 16 configured to carry out data processing such as an operation/determination on the basis of a detection result, discrimination data, and the like, and control section 17 (control means) configured to control an operation of each section.

Each of the apparatuses 11 to 14 is provided with a conveyor type transfer section 20 configured to transfer test tubes 25 upright along a predetermined transfer path 20a. The transfer paths 20a of the transfer sections 20 of the plurality of apparatuses 11 to 14 are continuously arranged.

As shown in FIGS. 2 to 5D, a test tube 25 serving as a sample container for containing therein a sample is supported upright on a holder 24, and is transferred by the movement of a transfer belt of the transfer section 20. The test tube 25 is, for example, a vacuum blood collection tube, is constituted of transparent glass or plastic, and is formed into a cylindrical shape having inside a cylindrical space for containing therein a sample. A label 27 is attached by adhesion to the outer surface of the test tube 25. A barcode 27a serving as a discrimination data indication section configured to indicate various data items such as discrimination data and the like of the sample 25a is shown on the label 27. As shown in FIG. 5A, inside the test tube 25, three layers including a blood clot 25b, silicone 25c serving as a separating agent, and blood serum 25d are contained as a sample 25a. It should be noted that in FIG. 5A, the label 27 is omitted to facilitate the explanation.

The carry-in apparatus 11 shown in FIG. 1 is provided with a transfer section 20, rack installation section 11a arranged at a lateral position of the transfer section 20, and transfer mechanism 11b configured to transfer the test tube 25 held on a rack set on the rack installation section 11a to a holder 24 of the transfer section 20.

As shown in FIGS. 2 and 3, the subdivision/dispensing apparatus 12 is constituted by being provided with an apparatus main body 31, the transfer section 20 provided on the apparatus main body 31, reading section 32 configured to acquire various data items such as discrimination data indicated by the barcode 27a shown on the label 27 attached to the outer surface of the test tube 25, position data of the barcode 27a, and the like, label exfoliation section 33 (label removal unit) configured to exfoliate part of the label 27 attached to the side part of the test tube 25, detection section 34 (detection unit) configured to detect a state of the sample 25a in the test tube 25, and subdivision/dispensing section 35 (subdivision/dispensing unit) configured to subdivide/dispense the sample 25a.

The reading section 32 is provided at a side part of the transfer path 20a, reads the barcode 27a of the label 27 attached to the outer surface of the test tube 25 to be sent by the transfer section 20, and acquires various data items on the sample.

As shown in FIGS. 4A and 4B, the label exfoliation section 33 is constituted by being provided with base sections 41 arranged on the transfer path 20a side, a pair of arm sections 42 extending from the base sections 41 toward the transfer path 20a, and supported movably, and a pair of scraping blades 43 provided at distal ends of the pair of arm sections, and configured to scrape off the label 27. The scraping blades 43 are moved downward in a state where the scraping blades 43 are in contact with the label 27, whereby part of the label 27 is scraped off, is exfoliated from the test tube 25, and the part of the label 27 is removed.

The detection section 34 is provided with an imaging section 45 (image detection means) provided at a lateral position of the transfer path 20a on the downstream side of the label exfoliation section 33. The imaging section 45 is constituted of, for example, a CCD camera, and images the side of the sample 25a from a lateral position of the test tube 25 held upright on the transfer path 20a to acquire image data. The acquired image data is sent to the storage section 15.

The subdivision/dispensing section 35 is provided with a raising and lowering mechanism section 51 provided at a lateral position of the transfer path 20a, and subdivision/dispensing chip 52 supported on the raising and lowering mechanism section 51. The subdivision/dispensing chip 52 is arranged in opposition to an upper part of the stopper body 26 located at an upper part of the test tube 25, and is provided upwardly/downwardly movable by the raising and lowering mechanism section 51. A distal end part of the subdivision/dispensing chip 52 is provided with a nozzle needle 53 extending downwardly, and serving as a penetration insertion section. A nozzle hole 53a of the nozzle needle 53 communicates with a suction/ejection device (not shown) configured to subdivide/dispense a sample such as blood or the like. It should be noted that a distal end (lower end) part of the nozzle needle 53 is formed into a sharp-pointed shape, and can be inserted into the inside of the test tube 25 penetratingly through the stopper body 26 when the nozzle needle is lowered.

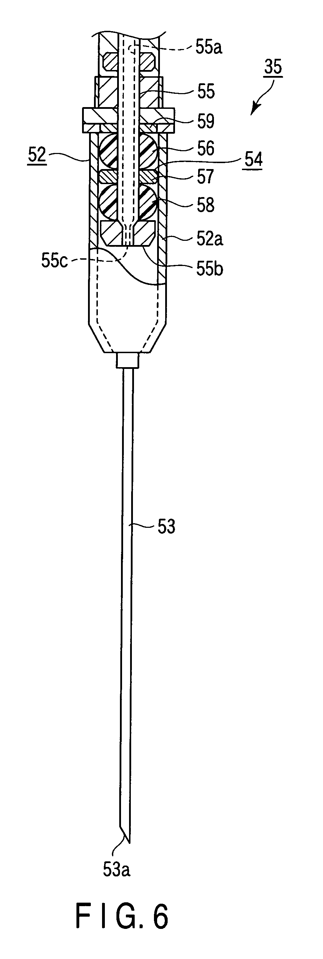

As shown in FIG. 6, a nozzle main body 54 is inserted into the subdivision/dispensing chip 52. The nozzle main body 54 is constituted by being provided with a fixed section 59 provided at an upper part thereof, subdivision/dispensing tube 55 configured to be axially movable direction within the subdivision/dispensing chip 52, and forming a path 55a inside, nozzle section 55b provided at a lower part of the subdivision/dispensing tube 55, further, first silicone ball 56, movable section 57, and second silicone ball 58 which are provided in sequence from above between the fixed section 59 and nozzle section 55b, and around the subdivision/dispensing tube 55.

The first and second silicone balls 56 and 58 and movable section 57 are loosely fitted on the subdivision/dispensing tube 55 movable axially. The nozzle section 55b is provided at a distal end of the subdivision/dispensing tube 55, and is moved together with the subdivision/dispensing tube 55. The nozzle section 55b includes a hole 55c communicating with the path 55a. The hole 55c is connected with the suction/ejection device (not shown) through the subdivision/dispensing tube 55. The nozzle main body 54 is connected to a raising and lowering mechanism (not shown), and fluid cylinder (not shown) constituting a ball compression mechanism.

The nozzle main body 54 before the subdivision processing is carried out is in a state where the subdivision/dispensing tube 55 is downwardly protruded, and nozzle section 55b and fixed section 59 are separated from each other, and the first and second silicone balls 56 and 58 are brought into a state where the balls 56 and 58 are really spherical (not shown). In this state, the overall nozzle main body 54 is moved by the raising and lowering movement mechanism to be positioned at an upper part of the subdivision/dispensing chip 52. Further, the overall nozzle main body 54 is lowered to be inserted into the inside of a cylinder section 52a of the subdivision/dispensing chip 52.

From this state, in a state where the fixed section 59 is fixed, the subdivision/dispensing tube 55 is moved upward by the fluid cylinder. Then, the nozzle section 55b upwardly moves to come close to the fixed section 59, and a space between the nozzle section 55b and fixed section 59 is narrowed. At the same time, the silicone balls 56 and 58 and movable section 57 are moved upward, and the silicone balls 56 and 58 are axially compressed. This state is shown in FIG. 6.

The silicone balls 56 and 58 are compressed axially, and are elastically expanded diametrically to be brought into close contact with the inner circumferential surface of the cylinder section 52a of the subdivision/dispensing chip 52. As a result of this, the nozzle main body 54 and subdivision/dispensing chip 52 are connected to each other in an airtight state.

In this state, the nozzle main body 54 and subdivision/dispensing chip 52 are moved by the raising and lowering movement mechanism in an integrated manner, the subdivision/dispensing chip 52 is inserted into the test tube 25, and thereafter when suction is carried out by the suction/ejection device, the pressure inside the subdivision/dispensing chip 52 becomes the negative pressure through the subdivision/dispensing tube 55, whereby it is possible to dispense the blood serum in the test tube into the subdivision/dispensing chip 52.

Furthermore, when the nozzle main body 54 and subdivision/dispensing chip 52 are moved upward by the raising and lowering movement mechanism in an integrated manner, the subdivision/dispensing chip 52 gets out of the test tube, and it is possible to position the nozzle main body 54 containing therein the dispensed blood serum, and subdivision/dispensing chip 52 at another test tube or the like. Further, when ejection is carried out by using the suction/ejection device, the pressure inside the subdivision/dispensing chip 52 becomes the positive pressure, and it is possible to dispense the blood serum in the subdivision/dispensing chip 52 into a test tube or the like.

Hereinafter, the processing procedure of the sample processing method of the sample processing unit 1 according to this embodiment will be described below.

First, a test tube 25 held on the rack 11c of the rack installation section 11a is transferred to a holder 24 on standby on the transfer section 20 by using the carry-in apparatus 11 shown in FIG. 1. The transferred test tube 25 is conveyed to a subdivision/dispensing apparatus 12 located on the downstream side in an upright position in which the test tube 25 is held by the holder 24 along the transfer path 20a.

Further, various data items such as the discrimination data indicated by the barcode 27a, position data of the barcode 27a, presence/absence of the label 27, and the like are detected (read processing) by the reading section 32 located on the downstream side. The acquired various data items are recorded on the storage section 15, and are used for the control to be carried out by the control section 17.

Furthermore, at the label exfoliation section 33 located on the downstream side, when the test tube 25 is arranged and stopped at the processing position, the scraping blades 43 are moved downward in a state where the blades 43 are in contact with the label 27, as shown in FIG. 4, whereby part of the label 27 is scraped off, is exfoliated from the test tube 25, and is removed from the test tube (label removal processing).

At the side part of the test tube 25 after the label removal processing, the label is scraped off in the parts 27b to be exfoliated extending vertically, and the transparent side part of the test tube 25 is partially in a exposed state. After the label removal processing is completed, the test tube 25 is sent to the detection section 34 and subdivision/dispensing section 35 which are located on the downstream side in an upright position in which the test tube 25 is held by the holder 24 along the transfer path 20a.

Subsequently, at the subdivision/dispensing section 35 shown in FIGS. 5A to 5D, a predetermined amount of the blood serum is dispensed from the test tube 25 containing therein the sample by using the subdivision/dispensing chip 52, and the blood serum is dispensed into a sample cup separately sent to the section 35 (subdivision/dispensing processing).

In carrying out the subdivision processing, the state of the test tube 25 or the sample 25a is detected by using the imaging section 45 of the detection section 34 provided at a lateral position (detection processing), and subdivision processing is controlled in accordance with the detection result. In the detection processing, the side part of the test tube 25 is firstly imaged to thereby acquire image data of the sample. In the side part of the test tube 25 sent to the imaging section 45, the label 27 has been partially exfoliated in advance by the label exfoliation section 33 located on the upstream side, and the transparent side wall of the test tube 25 is partially exposed, and hence it is possible to acquire image data on the sample 25a inside the test tube 25 through the transparent side wall of the test tube 25 from the lateral position. The acquired image data is recorded on the storage section 15, and is sent to the data processing section 16. The data processing section 16 detects (determines) the state of the sample 25a on the basis of the acquired image data. More specifically, the data processing section 16 detects positions of the blood clot surface 25e, silicone surface 25f, and blood serum surface 25g of the sample 25a, and nozzle needle 53 on the basis of the image data acquired by the imaging section 45 from the lateral position of the test tube 25. The control section 17 detects the position at which the distal end of the nozzle needle 53 is located by detecting the position of the nozzle needle 53, and positions of the liquid surfaces, and controls the movement of the nozzle in accordance with the detection result. By carrying out the positioning of the nozzle in such a manner that the distal end of the nozzle needle 53 stops at, for example, a position above the silicone surface 25f, it becomes possible to prevent the nozzle needle 53 from plunging into the silicone surface 25f, and subdivide the entirety of the blood serum 25d until nothing is left.

In the subdivision processing, the test tube 25 is firstly transferred along the transfer path 20a, as shown in FIG. 5A, and the transfer of the test tube 25 is stopped at a predetermined position directly under the nozzle needle 53, as shown in FIG. 5B. In this state, the subdivision/dispensing chip 52 is lowered by the raising and lowering mechanism section 51 to insert the nozzle needle 53 into the inside of the test tube 25 penetratingly through the stopper body 26, as shown in FIG. 5C. Furthermore, as shown in FIG. 5D, the distal end of the nozzle needle 53 is positioned in such a manner that the distal end thereof is at a position of a predetermined depth. At this time, the positioning is carried out in such a manner that the distal end of the nozzle needle 53 stops at, for example, a position above the silicone surface 25f by the above-mentioned detection/control. In this state, suction through the nozzle needle 53 is carried out by using the suction/ejection mechanism, whereby the entirety of the blood serum 25d is subdivided as the sample 25a until nothing is left (subdivision processing).

After the blood serum 25 has been subdivided, the subdivision/dispensing chip 52 is moved upward by the raising and lowering mechanism section 51, nozzle needle 53 is positioned at a sample cup separately sent to the nozzle needle 53, and ejection is carried out by the suction/ejection mechanism, whereby the sample is dispensed into the sample cup (dispensing processing). The sample cup is taken out of the carry-out apparatus 13 (carry-out processing), and is carried into the analysis apparatus 14 located on the downstream side. Further, analysis processing for testing various reactions is carried out by the analysis apparatus 14.

According to the sample processing unit 1 associated with this embodiment, the following advantages can be obtained. That is, it is possible to carry out the sample processing in the stopper-inserted state by using the nozzle needle 53 serving as a penetration insertion section which can be inserted into the test tube 25 penetratingly through the stopper body 26. Accordingly, it is not necessary to remove the stopper in advance, and hence it is possible to carry out the processing with a high degree of efficiency, prevent the sample from dispersing and evaporating, and prevent foreign matter from being mixed.

Further, it is possible to carry out the subdivision processing while detecting the state inside the test tube by using the detection section 34, and hence it becomes possible to subdivide the entirety of the blood serum 25d until nothing is left, and carry out control in order that the nozzle needle 53 may not intrude into the silicone surface 25f, whereby the processing accuracy is improved. Furthermore, by providing the label exfoliation section 33 on the upstream side of the detection section 34, even when the label 27 is attached to the side part of the test tube 25, it is possible to partially exfoliate and remove the label 27 in advance, and securely acquire the image data on the sample from the lateral position. As a result of this, it is possible to maintain a high degree of detection accuracy.

### [Second Embodiment]

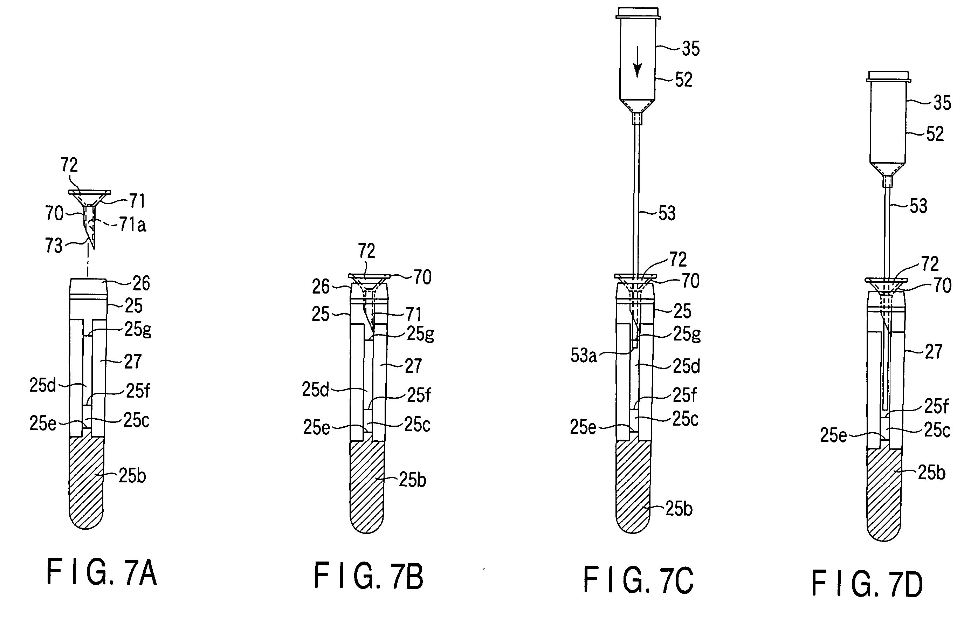

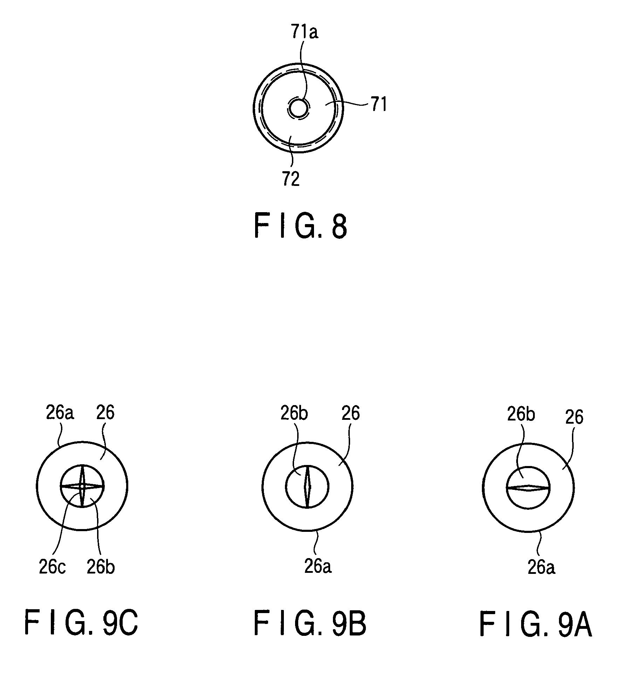

Hereinafter, a second embodiment of the present invention will be described below with reference to FIGS. 7A to 8. FIGS. 7A to 7D are explanatory views of a structure and subdivision processing of a subdivision/dispensing section 35 according to this embodiment. FIG. 8 is a plan view of a chip slope. It should be noted that this embodiment is identical to the first embodiment described previously except that a chip slope 70 is used in the subdivision processing, and hence a description common to the embodiments will be omitted.

In this embodiment, a subdivision/dispensing section 35 which is a penetration processing unit is constituted by being provided with a chip slope 70.

The chip slope 70 shown in FIGS. 7A to 7D, and 8 is constituted by being provided with a tubular section 71 having a diameter greater than a nozzle needle 53, and diameter-expansion section 72 continuing above and from the tubular section 71 both of which are provided in an integrated manner. The chip slope 70 is made of a resin, a distal end 73 (lower end) of the tubular section 71 is formed into an acute shape, and is formed to be able to penetrate a stopper body 26. The diameter-expansion section 72 has a conical shape formed in such a manner that an upper part thereof has a greater diameter, and lower part thereof has a smaller diameter. Here, it is assumed that the diameter of the nozzle needle 53 is, for example, 2 mm, and inner diameter of the tubular section 71 is, for example, 3.6 mm. As a result, it is possible to keep the opening area to a small value while enabling opening to the atmosphere. Further, a subdivision/dispensing chip 52 is made of a resin.

In a sample processing apparatus according to this embodiment, at the time of subdivision, the chip slope 70 is lowered from above a stopper body 26 prior to insertion of the nozzle needle 53, whereby the tubular section 71 penetrates the stopper body 26, and distal end 73 of the tubular section 71 is inserted into a test tube 25 to be arranged therein. In this state, the inside and outside of the test tube 25 communicate with each other through a cavity 71a inside the tubular section 71, and inside of the test tube is opened to atmospheric pressure.

Subsequently, the subdivision/dispensing chip 52 is lowered to insert the nozzle needle 53 from above the tubular section 71 into the section 71. The nozzle needle 53 is passed through the cavity 71a of the tubular section 71 to be inserted into the test tube 25. This state is a state where the inside of the test tube 25 communicates with the atmosphere through a part between an outer circumference of the nozzle needle 53 and inner surface of the cavity 71a. It should be noted that at this time, like in the first embodiment, a control section 17 detects the position at which the distal end of the nozzle needle 53 is located by detecting the position of the nozzle needle 53, and positions of the liquid surfaces, and controls the movement of the nozzle in accordance with the detection result. In this state, like in the first embodiment, a subdivision operation of subdividing the blood serum by using the subdivision/dispensing chip 52 is carried out. Other operations are also carried out in the same manner as the first embodiment.

In a sample processing apparatus according to this embodiment too, like in the first embodiment, it is possible to securely subdivide only the blood serum 25d by detecting the position of the distal end of the nozzle needle 53 from the lateral position of the test tube in the state where the label 27 is partially exfoliated. Furthermore, in this embodiment, the chip slope 70 is inserted in advance to make the inside of the test tube 25 communicate with the atmosphere, whereby it becomes possible to prevent the inside of the test tube from being evacuated by the subdivision operation, and carry out the subdivision work easily and accurately. Further, by using the chip slope having the tubular section 71 with a long and thin shape, and diameter-expansion section 72, it is possible to keep the area by which the inside of the test tube is opened to the atmosphere to a small value, and hence it is possible to maintain the function of preventing dispersion and evaporation. Furthermore, each of the subdivision/dispensing chip 52 and chip slope 70 is made of a resin, whereby throwaway use is enabled, and prevention of contamination is also enabled.

### [Third Embodiment]

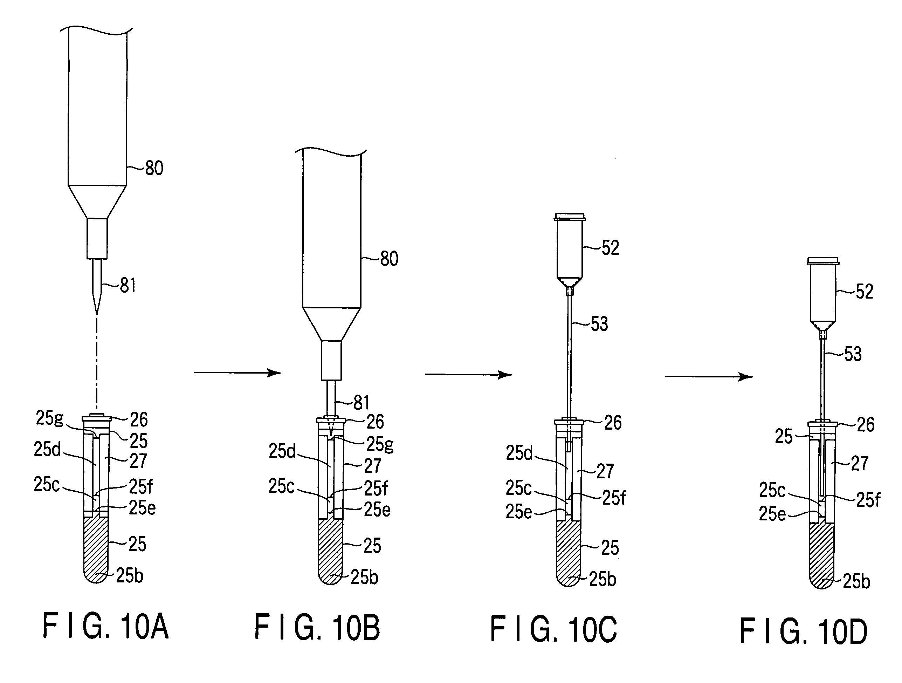

Hereinafter, a third embodiment of the present invention will be described below with reference to FIGS. 9A to 9C, and FIGS. 10A to 10D. FIGS. 9A to 9C are plan views of a stopper body according to this embodiment, and FIGS. 10A to 10D are explanatory views of a structure and subdivision processing of a subdivision/dispensing section 35 according to this embodiment. It should be noted that this embodiment is identical to the first embodiment or second embodiment described previously except that the structure of a stopper body 26 differs from the first or second embodiment, and that an ultrasonic cutter 80 is used, and hence a description common to the embodiments will be omitted.

As shown in FIGS. 9A to 9C, a stopper body 26 of this embodiment is a seal stopper, and is constituted by including an outer circumferential section 26a, and central section 26b both of which are formed integral with each other. The outer circumferential section 26a is constituted of, for example, a metal such as aluminum foil or the like, and central section 26b is constituted of, for example, rubber or the like.

In this embodiment, the subdivision/dispensing section 35 which is a penetration processing unit is constituted by being provided with an ultrasonic cutter 80. The ultrasonic cutter 80 has a blade 81 formed into a sharp-edged shape at a distal end thereof.

In a sample processing apparatus according to this embodiment, at the time of subdivision, the ultrasonic cutter 80 is lowered from above a stopper body 26 prior to insertion of a nozzle needle 53, as shown in FIGS. 9A and 9B, and central section 26b of the seal stopper body 26 is cut crosswise by the blade 81 at the distal end of the cutter 80, as shown in FIGS. 10A to 10D, thereby forming an opening 26c. For example, first, as shown in FIG. 10A, the section 26b is cut laterally, and then the ultrasonic cutter 80 is moved upward again. Subsequently, the sample 25 is rotated 90°, and ultrasonic cutter 80 is lowered again, thereby cutting the central section 26b longitudinally, as shown in FIG. 10B. By the operation described above, a cross-shaped opening 26c is formed as shown in FIG. 10C. The blade 81 is sharply edged, and hence it is possible to easily form an opening 26c with small pressure. As a result of the above, the inside and outside of the test tube 25 communicate with each other, and the inside of the test tube 25 is opened to atmospheric pressure.

Subsequently, as shown in FIGS. 9C and 9D, the subdivision/dispensing chip 52 is lowered to insert the nozzle needle 53 into the tubular section 71 from above the section 71. The nozzle needle 53 is passed through the cut cross-shaped opening 26c to be inserted into the test tube 25. It should be noted that at this time, like in the first and second embodiments, a control section 17 detects the position at which the distal end of the nozzle needle 53 is located by detecting the position of the nozzle needle 53, and positions of the liquid surfaces, and controls the movement of the nozzle in accordance with the detection result. In this state, like in the first embodiment described above, a subdivision operation of subdividing the blood serum by using the subdivision/dispensing chip 52 is carried out. Other operations are also carried out in the same manner as the first and second embodiments. It should be noted that in order to prevent contamination, the blade 81 of the ultrasonic cutter 80 is washed with a medical solution or water every time.

In a sample processing apparatus according to this embodiment too, like in the first embodiment, it is possible to securely subdivide only the blood serum 25d by detecting the position of the distal end of the nozzle needle 53 from the lateral position of the test tube in the state where the label 27 is partially exfoliated. Furthermore, according to this embodiment, by previously cutting the central section 26b included in the stopper body 26, the central section 26b being constituted of rubber, and forming an opening with the ultrasonic cutter 80 having the sharp blade 81, it becomes possible to prevent high pressure from being applied to the central section 26b, and carry out processing without damaging the outer circumferential section 26a constituted of aluminum foil.

It should be noted that the present invention is not limited to the above-mentioned embodiments as they are, and in the implementation stage, the constituent elements can be modified and embodied within the scope not deviating from the gist of the invention. For example, although the subdivision processing has been exemplified as the object of the processing, the processing contents are not limited to the above, and the present invention can also be applied to other processing items. Further, the processing object is not limited to that exemplified in each of the embodiments, and may be processing to be carried out for, for example, the sample container. Further, by appropriately combining a plurality of constituent elements disclosed in the embodiments described above with each other, various inventions can be formed. For example, some constituent elements may be deleted from all the constituent elements shown in the embodiments. Furthermore, constituent elements of different embodiments may be appropriately combined with each other.

## Claims

1. A sample processing unit (1) comprising:
a penetration processing unit (35) including a penetration insertion section (53) configured to be able to penetrate a stopper body (26) fitted in a sample container (25) in which a sample can be contained, and configured to subject the sample container (25) or the sample to processing;
a detection unit (34) configured to detect a state of the sample or the sample container (25) at the time of the processing; and
a label removal unit (33) configured to remove at least part of a label (27) attached to a side part of the sample container (25) prior to the detection.

2. The sample processing unit (1) according to claim 1, wherein
the sample container (25) is a test tube at least part of which is transparent,
the stopper body (26) is constituted of a material including rubber or synthetic resin material, and
the penetration processing unit (35) is a subdivision/dispensing unit including a nozzle needle (53) comprising a sharp distal end (53a), and configured to carry out subdivision processing or dispensing processing of the sample in a state where the distal end (53a) of the nozzle needle (53) is inserted into the sample container (25) penetratingly through the stopper body (26).

3. The sample processing unit (1) according to claim 1, wherein
the detection unit (34) acquires image data on the sample or the sample container (25), and detects at least any one of positions of surfaces of a blood serum, silicone, and a blood clot all of which serve as constituents of the sample contained in the sample container, and the penetration insertion section on the basis of the image data.

4. The sample processing unit (1) according to claim 1, further comprising a transfer unit (20) configured to transfer the sample container (25) along a predetermined transfer path (20a) while holding the sample container (25) upright with the stopper body (26) arranged on the upper side, wherein
the penetration processing unit (35) and the detection unit (34) are arranged along the transfer path (20a), and
the label removal unit (33) is arranged on the upstream side of the penetration processing unit (35) on the transfer path (20a).

5. The sample processing unit (1) according to claim 2, wherein
the penetration processing unit (35) further comprises a chip slope (70) including a tubular section (71) comprising a cavity with a diameter larger than the nozzle needle (53), and configured to penetrate the stopper body (26), and a diameter-expansion section (72) which continues from the tubular section (71), and the diameter of which is gradually expanded,
the chip slope (70) is inserted into the sample container (25) penetratingly through the stopper body (26) prior to the subdivision processing of the sample, and
the subdivision processing is carried out in a state where the inside and the outside of the sample container communicate with each other through the cavity of the chip slope (70).

6. The sample processing unit (1) according to claim 2, further comprising a cutter (80) configured to form an opening in the stopper body (26), wherein
an opening is formed in the stopper body (26) by using the cutter (80) prior to the subdivision processing of the sample.

7. A sample processing method of carrying out processing in a sample container (25) in which a sample can be contained, comprising:
a step of causing a penetration insertion section (53) capable of penetrating a stopper body (26) fitted in the sample container (25) to penetrate the stopper body (26) to insert the penetration insertion section (53) into the sample container (25); and
a step of carrying out processing in a state where the penetration insertion section (53) is inserted in the sample container (25).
